(19) Europäisches Patentamt
European Patent Office
Office européen des brevets

(11) Veröffentlichungsnummer: **0 030 632**
**B1**

(12) **EUROPÄISCHE PATENTSCHRIFT**

(45) Veröffentlichungstag der Patentschrift: **20.02.85**

(21) Anmeldenummer: **80107119.2**

(22) Anmeldetag: **17.11.80**

(51) Int. Cl.[4]: **C 07 D 277/74,**
C 07 D 417/12, A 61 K 31/425

# (54) Benzothiazolderivate, diese enthaltende pharmazeutische Zubereitungen und Verfahren zu ihrer Herstellung.

(30) Priorität: **13.12.79 DE 2950095**

(43) Veröffentlichungstag der Anmeldung:
**24.06.81 Patentblatt 81/25**

(45) Bekanntmachung des Hinweises auf die Patenterteilung:
**20.02.85 Patentblatt 85/08**

(84) Benannte Vertragsstaaten:
**AT BE CH DE FR GB IT LI NL SE**

(56) Entgegenhaltungen:
**EP-A-0 006 347**
**DE-C-1 255 484**

**Chemical Abstracts, Band 90, Nr. 23 4. Juni 1979 Columbus, Ohio, USA M. ARAKAWA et al. "Benzothiazolylthioalkanoic acid derivatives" Seite 663, Spalte 2, Abstract Nr. 186930q**
**J.Org.Chem.43,2697-2700 (1978)**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung**
**Frankfurter Strasse 250**
**D-6100 Darmstadt (DE)**

(72) Erfinder: **Doll, Thomas, Dr.**
**Niklas-Vogtstrasse 2**
**D-6500 Mainz (DE)**
Erfinder: **Schacht, Erich, Dr.**
**Maulbeerstrasse 5**
**D-6107 Reinheim 5 (DE)**
Erfinder: **Radunz, Hans-Eckart, Dr.**
**Hügelstrasse 13**
**D-6109 Mühltal 2 (DE)**
Erfinder: **Schulze, Ernst, Dr.**
**Auf der Hardt 73**
**D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 030 632 B1

**Beschreibung**

Die Erfindung betrifft neue Benzothiazolderivate der allgemeinen Formel I

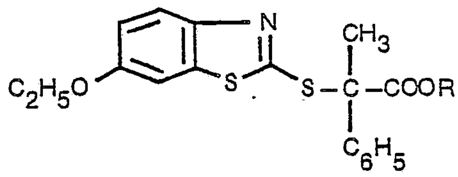

I

worin

R H oder Alkyl mit 1—4 C-Atomen bedeutet, sowie deren physiologisch unbedenkliche Salze.

Der Erfindung lag die Aufgabe zugrunde, neue Verbindungen mit wertvollen Eigenschaften aufzufinden, insbesondere solche, die Zur Herstellung von Arzneimitteln verwendet werden können. Diese Aufgabe wurde durch die bereitstellung der Verbindungen der Formel I gelöst.

Eine ähnliche Verbindung, α - Phenyl - 2 - benzothiazolylthioglykolsäure[=2 - (Benzothiazol - 2 - ylthio) - 2 - phenylessigsäure], ist in J. Org. Chem. *43*, 2697—2700 (1978) als Zwischenprodukt beschrieben. Weitere ähnliche, jedoch schwächer wirksame Verbindungen, insbesondere 2 - (Benzothiazol - 2 - ylthio) - 2 - methylpropionsäureäthylester, sind in der JP—A—79 03064 genannt (vgl. Chem. Abstr. *90*. 186930q (1979)).

Es wurde gefunden, daß die Verbindungen der Formel I und ihre physiologisch unbedenklichen Salze bei guter Verträglichkeit wertvolle pharmakologische Eigenschaften besitzen. Insbesondere zeigen sie antiarteriosklerotische und lipidspiegelsenkende Wirkungen. So worken sie sowohl cholesterinspiegelsenkend als auch triglyceridspiegelsenkend.

Die Bestimmung des Serumcholesterins und der Serumtriglyceride kann erfolgen durch eine vollautomatische, enzymatische Methode nach Christ, G.A. et al. (Technicon Symposium 1978, Frankfurt) in Anlehnung an die von Röschlau (Röschlau, P. et al (1975), 9th Int. Congr. on Clin. Chemistry, Toronto, Abstr. No. 1 angegebene Bestimmungsmethodik für Cholesterin und die von Wieland publizierte (H. U. Bergmeyer (Ed.), Methoden der enzymatischen Analyse, Chemie, Weinheim Bergstrasse, 1962) Triglyceridbestimmung.

Weiterhin wird das Verhältnis der α- und β-Lipoproteine im Sinne einer α-Lipoproteinerhöhung verschoben. Die Bestimmung der Lipoproteine kann erfolgen mit Hilfe einer von Kostner (Kostner, G. M. (1976), Clin. Chemistry 22, 5, 695) angegebenen polyanionischen Methodik und anschließender Bestimmung des HDL-Cholesterins wie oben angegeben.

Die Verbindungen der Formel I können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Gegenstand der Erfindung sind die Verbindungen der Formel I sowie ihre physiologisch unbedenklichen Salze.

Die Verbindungen der Formel I besitzen in der Seitenkette ein asymmetrisches Kohlenstoffatom. Daher können sie als Racemate sowie in verschiedenen optisch aktiven Formen vorliegen.

Gegenstand der Erfindung ist ferner ein Verfahren zur Herstellung der Verbindungen der Formel I sowie ihrer physiologisch unbedenklichen Salze, dadurch gekennzeichnet, daß man

eine Verbindung der allgemeinen Formel II

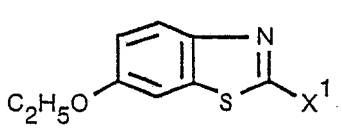

II

mit einer Verbindung der allgemeinen Formel III

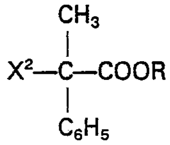

III

worin

der eine der beiden Reste $X^1$ und $X^2$ SH oder reaktionsfähig funktionell abgewanteltes SH,

der andere dieser beiden Reste | Cl, Br, J, OH oder reaktionsfähig funktionell abgewandeltes OH

bedeutet und

R | die bei Formel I angegebene Bedeutung hat

umsetzt und daß man gegebenenfalls einen erhaltenen Ester verseift und/oder eine erhaltene Säure verestert und/oder eine erhaltene Säure durch Behandeln mit einer Base in eines ihrer physiologisch umbedenklichen Metall- oder Ammoniumsalze umwandelt.

Die Herstellung der Verbindungen der Formel I erfolgt im übrigen nach an sich bekannten Methoden, wie sie in der Literatur (z.B. in den Standardwerken wie Houben-Weyl, Methoden der organischen Chemie, Georg Thieme-Verlag, Stuttgart; Organic Reaktions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formeln II und III können gewünschtenfalls auch in situ gebildet werden, derart, daß man sie aus dem Reaktionsgemisch nicht isoliert, sondern sofort weiter zu den Verbindungen der Formel I umsetzt.

In den Resten $X^1$ bzw. $X^2$ kann die SH-Gruppe funktionell abgewandelt sein, vorzugsweise in Form eines mercaptids, insbesondere eines Metallmercaptids, z.B. eines Alkalimetall-, Erdalkalimetall-, oder Schwermetall-mercaptids, vorzugsweise in Form des Natrium-, Kalium-, Silber-, Blei-, Zink- oder Quecksilber-mercaptids. In den Resten $X^1$ bzw. $X^2$ kann die OH-Gruppe funktionell abgewandelt sein, vorzugsweise in Form eines reaktionsfähigen Esters, z.B. eines Alkylsulfonats (worin die Alkylgruppe insbesondere 1—6 C-Atome hat, z.B. Methansulfonyloxy) oder eines Arylsulfonats (worin die Arylgruppe insbesondere 6—10 C-Atome hat, z.B. Benzolsulfonyloxy, p-Toluolsulfonyloxy, 1- oder 2-Naphthalinsulfonyloxy).

Die Ausgangsstoffe der Formeln II und III sind teils bekannt, teils neu. Die nicht bekannten unter diesen Ausgangsstoffen lassen sich jedoch in Analogie zu den bekannten Ausgangsstoffen nach an sich bekannten Methoden herstellen. So sind z.B. die 2-Halogenbenzothiazole der Formel II ($X^1$ = Cl, Br oder J) erhältlich durch Halogenierung der entsprechenden Benzothiazole (entsprechend Formel II, aber H an Stelle von $X^1$) oder der entsprechenden Phenylsenföle mit z.B. $PCl_5$ oder $PBr_5$. Die 2-Halogenpropionsäurederivate der Formel III ($X^2$ = Cl, Br oder J) sind ähnlich durch Halogenierung der zugrunde liegenden Propionsäurederivate (entsprechend III, aber H an Stelle von $X^2$) zugänglich. Die entsprechenden Mercaptoverbindungen der Formeln II ($X^1$ = SH) bzw. III ($X^2$ = SH) sind aus den Halogenverbindungen durch Reaktion mit NaSH herstellbar.

In der Regel arbeitet man bei der Umsetzung von II mit III unter Zusatz einer Base, z.B. eines Metalloxids wie Silber-, Blei-, Zink-, Quecksilber- oder Calciumoxid; eines Metallhydroxids, insbesondere eines Alkalimetall- oder Erdalkalimetallhydroxids, wie NaOH, KOH, LiOH oder $Ca(OH)_2$; eines Alkali- oder Erdalkalimetallcarbonats, z.B. $Na_2CO_3$ oder $K_2CO_3$, eines Alkali- oder Erdalkalimetallhydrids, z.B. NaH oder KH; eines Alkali- oder Erdalkalimetallalkoholats, z.B. Natrium- oder Kaliummethylat, Natrium- oder Kaliumäthylat, Kalium-tert.-butylat; einer organischen Base, z.B. Triäthylamin oder Benzyltrimethylammoniumhydroxid. Grundsätzlich eignen sich alle salzbildenden (mercaptidbildenden) Basen. Als Zwischenprodukt bei der Umsetzung der Mercaptoverbindung II ($X^1$ = SH) bzw. III ($X^2$ = SH) mit der Base bildet sich in der Regel das entsprechende mercaptid. Verwendet man als Reaktionspartner eine Halogencarbonsäure der Formel III ($R^3$ = OH), so wird auch diese vorzugsweise in Form eines ihrer Salze (z.B. des Na-, K-, Li- oder Ba-Salzes) in die Reaktion eingesetzt.

Die Umsetzung von II mit III kann in Absesenheit oder vorzugsweise in Gegenwart eines inerten Lösungs- bzw. Suspensionsmittels erfolgen. Als solche eignen sich z.B. Kohlenwasserstoffe, wie Benzol, Toluol oder Xylol; Alkohole, wie Methanol, Äthanol, Isopropanol, n-Propanol, n-Butanol oder tert.-Butanol; Äther, wie Diäthyläther, Diisopropyläther, Tetrahydrofuran (THF), Dioxan oder Diäthylenglykoldimethyläther; Amide wie Acetamid, Dimethylformamid (DMF); Nitrile wie Acetonitril; Sulfoxide wie Dimethylsulfoxid; Wasser; ferner Gemische der genannten Lösungsmittel. Die Reaktion wird zweckmäßig bei Temperaturen zwischen etwa 0 etwa 200°, vorzugsweise zwischen 20 und 150° durchgeführt; die Reaktionsdauer liegt zwischen etwa 120 minuten und mehreren Tagen, je nach den angewendeten Bedingungen. Falls man ohne Lösungsmittel arbeitet, etwa beim Zusammenschmelzen eines Natrium-mercaptids II ($X^1$ = SNa) mit einem Bromcarbonsäuresalz (z.B. der Formel $Br—C(CH_3)(C_6H_5)—COONa$), kommen auch höhere Temperaturen in Betracht, bis etwa 300°. Das Arbeiten unter einem Inertgas wie Stickstoff oder Argon kann vorteilhaft sein.

Gewünschtenfalls kann ein erhaltener Ester der Formel I (R = Alkyl mit 1—4 C-Atomen) nach an sich bekannten Methoden zur entsprechenden Säure (I, R = H) verseift werden, vorzugsweise durch Hydrolyse in alkalischem Medium, z.B. mit NaOH oder KOH in einem Alkohol wie Methanol, Äthanol oder Isopropanol, falls erwünscht unter Zusatz von Wasser, bei Temperaturen zwischen etwa 0 und etwa 100, vorzugsweise zwischen 20 und 80°.

Eine erhaltene Säure der Formel I (R = H) kann nach in der Literatur beschriebenen methode verestert werden, beispielsweise indem man sie mit einem Alkohol der Formel R—OH (R = Alkyl mit 1—4 C-Atomen) umsetzt, vorzugsweise in Gegenwart einer anorganischen oder organischen Säure, wie HCl, HBr, HJ, $H_2SO_4$, $H_3PO_4$, Trifluoressigsäure, Benzolsulfonsäure oder p-Toluolsulfonsäure, oder eines sauren Ionenauschers in Gegenwart oder Abwesenheit eines inerten Lösungsmittels, wie z.B. Benzol, Toluol oder Xylol, bei Temperaturen zwischen etwa 0° und etwa 150°. Der Alkohol wird bevorzugt im Überschuß eingesetzt. Weiterhin kann man in Gegenwart wasserbindender Agentien arbeiten, z.B. von wasserfreien Schwermetallsalzen (wie $CuSO_4$ oder $ZnCl_2$) oder von Molekularsieben. Mann kann auch das Reaktionswasser azeotrop entfernen, wobei man vorteilhaft Kohlenwasserstoffe (z.B. Benzol oder Toluol) oder chlorierte Kohlenwasserstoffe (z.B. Chloroform oder 1,2-Dichloräthan) zusetzt. Unter milden Bedingungen verläuft die Veresterung, wenn man das Reaktionswasser chemisch durch Zusatz vorzugsweise äquimolarer Mengen an Carbodiimiden (z.B. N,N'-Dicyclohexylcarbodiimid) bindet, wobei man inerte Lösungsmittel wie Äther, Dioxan, Benzol oder 1,2-Dimethoxyäthan verwenden und Basen wie Pyridin zusetzen kann. Die Methylester (bzw. Äthylester) können auch durch Umsetzen der freien Säuren mit Diazomethan (bzw. Diazoäthan) in einem inerten Lösungsmittel wie Äther, Benzol oder Methanol hergestellt werden.

Weiterhin kann man Ester herstellen durch Umsetzen von Metallsalzen, vorzugsweise der Alkalimetall-, Blei- oder Silbersalze der säure mit Alkylhalogeniden, die dem betreffenden Alkohol entsprechen, gegebenenfalls in einem inerten Lösungsmittel, z.B. Äther, Benzol oder Petroläther.

Racemate der Formel I können nach Methoden, wie sie in der Literatur beschrieben sind, in ihre optischen Antipoden getrennt werden. Die Carbonsäure der Formel I (R = H) kann z.B. mit optisch aktiven Aminen wie Chinin, Brucin oder Strychnin in diastereomere Salze umgewandelt werden, die durch Kristallisation getrennt und hydrolytisch Aufgespalten werden können.

Eine erhaltene Säure kann durch Umsetzung mit einer Base in eines ihrer physiologisch unbedenklichen Metall- bzw. Ammoniumsalze übergeführt werden. Als Salze kommen insbesondere die natrium-, Kalium-, Magnesium-, Calcium- und Ammoniumsalze in Betracht, ferner substituierte Ammoniumsalze, z.B. die Dimethyl-, Diäthyl- und Diisopropylammonium-, Monoäthanol-, Diäthanol- und Triäthanolammonium-, Cyclohexylammonium-, Dicyclohexylammonium- und Dibenzyläthylen diammonium- salze.

Umgekehrt kann die Säure der Formel I aus ihren Metall- und Ammoniumsalzen durch Behandlung mit Säuren in Freiheit gesetzt werden.

Gegenstand der Erfindung ist ferner die Verwendung der Verbindungen der Formel I zur Herstellung pharmazeutische Zubereitungen, insbesondere auf nicht-chemischem Wege. Hierbei könnensie (oder eines ihrer physiologisch unbedenklichen Salze) zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner pharmazeutische Zubereitungen, gekennzeichnet durch einen Gehalt an mindestens einer Verbindung der Formel I und/oder einem ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyäthylenglykole, Glycerintriacetat, Gelatine, Kohlehydrate wie Lactose oder Stärke, Magnesiumstearat, Talk, Vaseline. Zurr oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder.

Sofern die Arzneimittel in Form abgeteilter Pulver verabreicht werden sollen, sind auch die Verpackungsmaterialien wie Papierbriefchen oder Papierkapseln geeignete Trägerstoffe. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Ge- schmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I können bei der therapeutischen Behandlung des menschlichen Körpers sowie bei der Bekämpfung von Krankheiten verwendet werden. Insbesondere eignen sie sich zur Behandlung und/oder Prophylaxe von Krankheitsbildern mit erhöhten Werten der Serumlipide und/oder Lipoproteinverschiebungen zu Gunsten von LDL- und/ oder VLDL-Fraktion, Von primären und sekundären Hyperlipoproteinämien mit und ohne Xanthomatose, von Atherosklerose (Koronarsklerose, Zerebralsklerose, periphere Gefäßsklerose), von diabetischen Angiopathien (diabetische Retinopathie).

Dabei werden die erfindungsgemäßen Substanzen in der Regel in Analogie zu bekannten, im Handel befindlichen Lipidsenkern (z.B. Clofibrat) verabreicht, vorzugsweise in Dosierungen zwischen etwa 10 und 1000 mg, insbesondere zwischen 50 und 500 mg pro Dosierungseinheit. Die tägliche Dosierung liegt

vorzugsweise zwischen etwa 0,2 und 100 mg/kg Körpergewicht. Die besondere Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, dem Geschlecht, von der Kost, vom Verabfolgungszeitpunkt und -weg, von der Ausscheidungsgeschwindigkeit, von Arzneistoffkombinationen und der Schwere der jeweiligen Erkrankung, welcher die Therapie gilt. Die orale Applikation ist bevorzugt.

Jede der in den folgenden Beispielen genannten Einzelverbindungen der Formel I ist zur Herstellung pharmazeutischer Zubereitungen besonders geeignet.

In den folgenden Beispielen bedeutet "übliche Aufarbeitung": Man gibt, falls erforderlich, Wasser hinzu, extrahiert mit Äther, trennt ab, trocknet die organische Phase über Natriumsulfat, filtriert, dampft ein und reinigt durch Chromatographie (an Kieselgel) und/oder Kristallisation.

Beispiel 1

Man löst 2,3 g Na in 80 ml Äthanol, gibt 21,1 g 6-Äthoxy-2-mercaptobenzothiazol hinzu und rührt bis zur Lösung Dann gibt man 25,7 g 2-Brom-2-phenylpropionsäureäthylester hinzu, rührt über Nacht bei 20°, arbeitet wie üblich auf und erhält 2-(6-Äthoxybenzothiazol-2-ylthio)-2-phenyl-propionsäureäthylester, F. 92—94°.

Beispiel 2

Man kocht ein Gemisch von 21,4 g 2-Chlor-6-äthoxybenzothiazol, 22,6 g Dinatriumsalz der 2-Mercapto-2-phenylpropionsäure und 200 ml n-Butanol 4 Stunden, dampft ein, arbeitet wie üblich auf und erhält 2-(6-Äthoxybenzothiazol-2-ylthio)-2-phenyl-propionsäure, F. 152—153°.

Beispiel 3

Man rührt 6 g 2-(6-Äthoxybenzothiazol-2-ylthio)-2-phenylpropionsäureäthylester und 6 g KOH in 100 ml Äthanol bei 20°, dampft ein, löst in Wasser, wäscht mit Äther, säuert mit Salzsäure an, extrahiert mit $CH_2Cl_2$, trennt ab, trocknet, dampft ein und erhält 2-(6-Äthoxy-benzothiazol-2-ylthio)-2-phenyl-propionsäure. F. 152—153° (aus $CCl_4$).

Beispiel 4

Man läßt eine Lösung von 1 g 2-(6-Äthoxy-benzothiazol-2-ylthio)-2-phenylpropionsäure in 15 ml äthanolischer Salzsäure 24 Stunden bei 20° stehen, dampft ein, arbeitet wie üblich auf und erhält 2-(6-Äthoxy-benzothiazol-2-ylthio)-2-phenylpropionsäureäthylester, F. 92—94°.

Beispiele 5 bis 10

Analog Beispiel 4 erhält man aus der Säure durch Reaktion mit den ensprechenden Alkoholen:

5. 2-(6-Äthoxybenzothiazol-2-ylthio)-2-phenyl-propionsäure-methylester.
6. 2-(6-Äthoxybenzothiazol-2-ylthio)-2-phenyl-propionsäure-propylester.
7. 2-(6-Äthoxybenzothiazol-2-ylthio)-2-phenyl-propionsäure-isopropylester.
8. 2-(6-Äthoxybenzothiazol-2-ylthio)-2-phenyl-propionsäure-butylester.
9. 2-(6-Äthoxybenzothiazol-2-ylthio)-2-phenyl-propionsäure-isobutylester.
10. 2-(6-Äthoxybenzothiazol-2-ylthio)-2-phenyl-propionsäure-sek.-butylester.

Die nachstehenden Beispiele betreffen pharmazeutische Zubereigungen, die Verbindungen der Formel I enthalten:

Beispiel A

*Tabletten*

Ein Gemisch von 1 kg 2-(6-Äthoxybenzothiazol-2-ylthio)-2-phenylpropionsäure, 4 kg Lactose, 1,2 kg kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B

*Dragees*

Analog Beispiel A werden Tabletten gepreßt, die anschließend in üblicher Weise mit einem Überzug aus Saccharose, kartoffelstärke, Talk, Tragant und Farbstoff überzogen werden.

Beispiel C

*Kapseln*

2 kg 2-(6-Äthoxybenzothiazol-2-ylthio)-2-phenyl-propionsäure werden in üblicher Weise in Hartgelatine-kapseln gefüllt, so daß jede Kapsel 20 mg des Wirkstoffs enthält.

Analog sind Tabletten, Dragees und Kapseln erhältlich, die einen oder mehrere der übrigen Wirkstoffe der Formel I und/oder ihrer physiologisch unbedenklichen Salze enthalten.

**Patentansprüche**

1. Benzothiazolderivate der allgemeinen Formel I

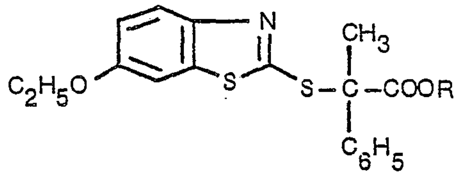

I

worin

R H oder Alkyl mit 1—4 C—Atomen bedeutet, sowie deren physiologisch unbedenkliche Salze.

2. 2-(6-Äthoxybenzothiazol-2-ylthio)-2-phenyl-propionsäure.

3. Verfahren zur herstellung von Benzothiazolderivaten der allgemeinen Formel I

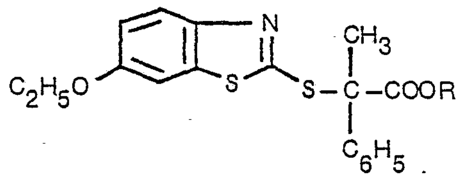

I

worin

R H oder Alkyl mit 1—4 C—Atomen bedeutet, sowie von deren physiologisch umbedenklichen Salzen, dadurch gekennzeichnet, daß man eine Verbindung der allgemeinen Formel II

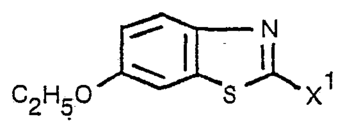

II

mit einer Verbindung der allgemeinen Formel III

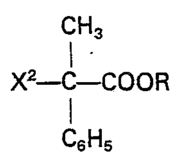

III

worin

| | |
|---|---|
| der eine der beiden Reste $X^1$ und $X^2$ | SH oder reaktionsfähig funktionell abgewanteltes SH, |
| der andere dieser beiden Reste | Cl, Br, J, OH oder reaktionsfähig funktionell abgewandeltes OH |
| bedeutet und R | die bei Formel I angegebene Bedeutung hat |

umsetzt

und daß man gegebenenfalls einen erhaltenen Ester verseift und/oder eine erhaltene Säure verestert und/oder eine erhaltene Säure durch Behandeln mit einer Base in eines ihrer physiologisch umbedenklichen Metall- oder Ammoniumsalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Salze.

**Revendications**

1. Dérivés de benzothiazol de formule générale I

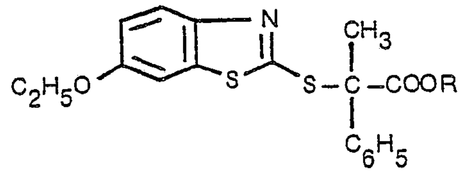

I

où

R représente H ou un alcoyle en $C_1$ à $C_4$, ainsi que leurs sels physiologiquement acceptables.

b 2. Acide 2-(6-éthoxy-benzothiazol-2-ylthio)-2-phényl-propionique.

3. Procédé de préparation de dérivés de benzothiazole de formule générale I

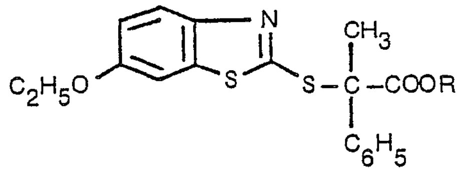

I

où

R représente H ou un alcoyle en $C_1$ à $C_4$, ainsi que de leurs sels physiologiquement acceptables, caractérisé en ce qu'on fait réagir un composé de formule générale II

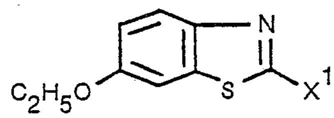

II

avec un composé de formule générale III

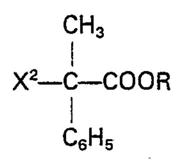

III

où

l'un des deux radicaux $X^1$ et $X^2$ représente SH ou SH fonctionnellement modifié de façon réactive, l'autre de ces deux radicaux représente Cl, Br, I, OH ou OH fonctinnellement modifié de façon réactive et R a la signification donnée pour la formule I, et en ce que le cas échéant on saponifie un ester obtenu et/ou on estérifie un acide obtenu et/ou on transforme un acide obtenu par traitement avec une base en l'un de ses sels métalliques ou d'ammonium physiologiquement acceptables.

4. Procédé de préparation de préparations pharmaceutiques, caractérisé en ce qu'on met sous une forme posologique appropriée un composé de formule I et/ou un de ses sels physiologiquement acceptables avec au moins un support ou additif solide, liquide ou semi-liquide et éventuellement en combinaison avec une ou plusieurs autres substances actives.

5. Préparation pharmaceutique caractérisée en ce qu'elle contient au moins un composé de formule générale I et/ou un de ses sels physiologiquement acceptables.

**Claims**

1. Benzothiazole derivatives of the general formula

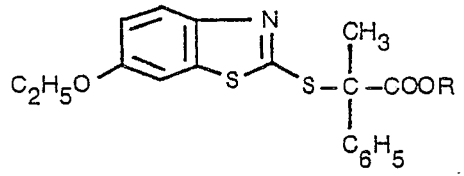

I

wherein R is H or alkyl having 1—4 C atoms, and physiologically acceptable salts thereof.

2. 2-(6-Ethoxy-benzothiazol-2-ylthio-2-phenyl-propionic acid.

3. Process for the preparation of benzothiazole derivatives of the general formula I

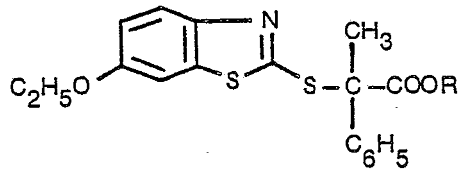

I

wherein R is H or alkyl having 1—4 C-atoms, and of physiologically acceptable salts thereof, characterised in that a compound of the general formula II

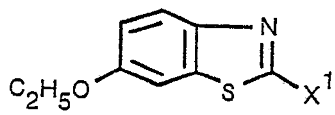

II

is reacted with a compound of the general formula III

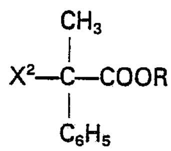

III

wherein one of the two radicals $X^1$ and $X^2$ is SH or SH which has been functionally modified so as to be reactive, the other of these two radicals is Cl, Br, I, OH or OH which has been functionally modified so as to be reactive, and R has the meaning indicated in formula I, and, if appropriate, a resulting ester is saponified and/or a resulting acid is esterified and/or a resulting acid is converted, by treatment with a base into one of its physiologically acceptable metal or ammonium salts.

4. Process for the preparation of pharmaceutical formulations, characterised in that a compound of the formula I and/or one of its physiologically acceptable salts is brought into a suitable dosage form together with at least one solid, liquid or semi-liquid excipient or adjuvant and, if appropriate, in combination with one or more further active compounds.

5. Pharmaceutical formulation characterised in that it contains at least one compound of the general formula I and/or one of its physiologically acceptable salts.